# EUROPÄISCHE PATENTANMELDUNG

(11) **EP 1 296 137 A2**
(43) Veröffentlichungstag der Anmeldung: **26.03.2003**
(21) Anmeldenummer: 02021444.1
(22) Anmeldetag: 25.09.2002
(51) Int. Cl.: G01N 33/10, A21C 9/00

(54) **Teig-Messung zur Überwachung und Regelung der Teigqualität bei der automatischen Teigbearbeitung**

(30) Priorität: 25.09.2001 DE 10146998
(71) Anmelder: A. FRITSCH GMBH & CO. KG, D-97348 Markt Einersheim (DE)
(72) Erfinder: Zauter, Georgi, 97348 Willanzheim (DE); Danzig, Gerhard, 97346 Iphofen-Mönchsondheim (DE); Wehner, Hans Joachim, 49733 Haren (DE)
(74) Vertreter: Götz, Georg, Dipl.-Ing.

(57) **Zusammenfassung**

Messverfahren zur Bestimmung und Überwachung der Teigqualität für eine automatische Teigbearbeitung, wozu mittels mindestens eines den Teig (11) kontaktierenden Druck- oder Kraft-Sensors (12) eine vom Teig (11) ausgeübte Reaktionskraft (F) oder Reaktionsauslenkung als Maß für dessen Festigkeit gemessen und entsprechende Messdaten oder -signale einer Steuerung für die Teigbearbeitung zugeleitet werden, wobei mehrere zeitlich und örtlich diskrete Messungen an fertig bereitetem Teig (11) ausgeführt werden, der als kontinuierliches Teigband oder als einzelne Teigstücke (11) ausgebildet ist, wobei das Teigband oder die Teigstücke (11) zumindest zeit -oder taktweise über ein oder mehrere Fördermittel (2a) in einer Förderrichtung (10) bewegt werden, und der Sensor (12) synchron zur Fördergeschwindigkeit mit dem Teig (11) in Kontakt gebracht wird.

## Beschreibung

Die Erfindung betrifft ein Meßverfahren zur Bestimmung und Überwachung der Teigqualität für eine automatische Teigbearbeitung gemäß Oberbegriff des Anspruchs 1. Ferner betrifft die Erfindung ein Verfahren zur Regelung und/oder Steuerung eines Prozesses des Teigformens/-aufbereitens gemäß Oberbegriff des Anspruchs 10, wobei das vorgenannte Meßverfahren Verwendung finden kann. Weiter betrifft die Erfindung eine Meßanordnung zur Bestimmung und Überwachung der Teigqualität für eine automatische Teigbearbeitung gemäß Oberbegriff des Anspruchs 16 und zur Durchführung des vorgenannten Meßverfahrens. Weiter betrifft die Erfindung eine Anordnung zur Regelung und/oder Steuerung eines Prozesses des Teigformens/-aufbereitens gemäß Oberbegriff des Anspruchs 25, wobei die vorgenannte Meßanordnung Einsatz finden kann.

Aus US-A-5 479 850 ist eine Vorrichtung und ein Verfahren zur maschinellen Herstellung von Brot bekannt. Aus einem Kornspeicher für unterschiedliche Getreidesorten wird rechnergesteuert entsprechend einem vorprogrammierten Rezept ein ausgewählter Speicherauslaß zu einem Mahlwerk automatisch angesteuert. Letzterem ist eine Mischkammer für das resultierende Mehl nachgeordnet. Der Mischkammer werden, ebenfalls rechnergesteuert gemäß vorprogrammiertem Rezept, flüssige und feste Ingredienzen sowie Wasser zugeführt. Der Rechner ist über eine Steuerungsleitung mit der Mischkammer gekoppelt und bestimmt den Umfang bzw. das Ausmaß, mit welchem gemischt wird. Über eine nachgeordnete Waage (ebenfalls mit dem Rechner gekoppelt) gelangt der Teig zur weiteren Formung in einen rechnergesteuerten Extruder, woraus einzelne Teigportionen abgeteilt werden. Diese werden an einen Gärschrank weitergeleitet, der mittels des Rechners temperaturgeregelt ist. Letzteres gilt auch für den nachgeordneten Backofen. Mittels einer Eingabetastatur kann im Rechner das gewünschte Rezeptprogramm angewählt werden.

Aus EP-A-0 191 288 ist ein Verfahren und eine Vorrichtung zur Teigbereitung für Brot oder Feingebäck bekannt. Zur Vermeidung subjektiver Teig-Beurteilungen und zu einer schnellen, präzisen und zuverlässigen Ermittlung der Teigqualität im Zuge des Mischens und Knetens von Teig wird dessen Zustand mit einer Meßsonde überwacht. Aus den Meßwerten, die zu ganz bestimmten charakteristischen Meßzeitpunkten erhalten werden, werden Zugabewerte für die weitere dosierte Zugabe von Hauptbestandteilen wie Mehl oder Wasser ermittelt. Eine hierzu vorgesehene Vorrichtung enthält die Meßsonde für die Aufnahme der Misch- bzw. Teigbeschaffenheit und einen digitalen Auswerterechner, der seinerseits auf eine Dosiereinrichtung für die Zugabe von Hauptbestandteilen wie z. B. Wasser einwirkt. Der Auswerterechner ist aber auch zur Steuerung bzw. Regelung der Wasserzugabetemperatur, der Werkzeugdrehzahl und weiterer Maschinenparameter vorgesehen. Die Meßsonde ist zur Aufnahme sowohl des Mittelwerts der vom Teig im Zuge seines Mischens ausgeübten Reaktionskräfte als auch des Anteils der schnell schwankenden Reaktionskräfte ausgebildet. Dadurch kann das Meßsignal in mehrere charakteristische Kennwerte wie Niederfrequenz- bzw. Gleichanteil und auch höher frequente Anteile aufgeschlüsselt werden. Eine Teigmasse soll über eine derartige Sonde aufschlußreiche höherfrequente Signale liefern können. Diese sollen sehr gut für die Steuerung der Teigbereitung verwertbar sein, insbesondere sollen sie sich für die benötigte Mischungsdauer heranziehen lassen. Weiterhin wird die Aufnahme eines Temperatursignals aus dem Teig als wichtiger Parameter für den Teigzustand vorgeschlagen. Durch Soll-/lstwertvergleiche lassen sich Werte zur Dosierung einer Ergänzungsmenge für einen Teig-Hauptbestandteil ermitteln und auch die weitere Mischungsdauer steuern.

DE-A-198 19 633 beschreibt das Wirken und Auswalzen von quer zu ihrer Längsrichtung geförderten Teigsträngen, wobei in eine Wirkplatte Sensoren zur Erfassung der Länge der gewickelten Teigstränge integriert sind. Mittels einer Sensor-Auswerteschaltung wird die Teiglänge zum einen angezeigt und zum anderen zum Ansteuern eines motorischen Verstellantriebs verwendet, mit welchem die Wirkplatte nach oben oder unten verstellt werden kann.

DE-A-31 23 195 beschreibt, wie anhand einer Strommessung für einen Kalibrierwalzenantrieb auf das dafür notwendige Drehmoment und daraus resultierend auf die Eingangsdicke des zu kalibrierenden Teigbandes geschlossen wird. Mittels eines Soll-/Istwertvergleichs und eines nachgeschalteten Reglers wird die Teigzuführung mittels Zuführwalzen bzw. deren entsprechenden Antrieb reguliert.

Eine Aufgabe der Erfindung besteht darin, dem im Oberbegriff des Anspruchs 1 genannten Teigbearbeitungs-Meßverfahren über den Prozeß des Teigknetens bzw. -mischens hinaus weitere Einsatzfelder insbesondere bei der maschinellen Serienproduktion backfertig aufbereiteter Teiglinge zu erschließen. Zur Lösung dieser Aufgabe wird das im Patentanspruch 1 angegebene Meßverfahren vorgeschlagen. Vorteilhafte Ausgestaltungen und Einzelmerkmale ergeben sich aus den abhängigen Ansprüchen.

Durch die erfindungsgemäß zeitlich und örtlich diskret vorzunehmenden Festigkeitsmessungen am Teigband oder an einzelnen Teigstücken läßt sich bereits mit wenigen Stichproben ein verhältnismäßig zuverlässiges Bild über den tatsächlichen Teigzustand, welcher dem Teigformen und -aufbereiten zur Verfügung steht, machen. Ein Teigdurchsatz und eine Produktionsgeschwindigkeit in jeweils ausreichendem Maß lassen sich durch die ständige Förderung des Teiges gewährleisten, die entweder kontinuierlich (ohne Unterbrechungen) oder auch taktweise (mit zeitlich möglichst kurzen Unterbrechungen) im Rahmen der Erfindung erfolgen kann. Die Zuverlässigkeit der Messung der Teigfestigkeit wird erfindungsgemäß dadurch erhöht, dass der Kraft- oder Drucksensor mit dem Teig synchron zu dessen Fördergeschwindigkeit in Kontakt gebracht wird. Ein weiterer, mit der Erfindung erzielbarer Vorteil besteht darin, dass relativ einfache Prinzipien der Kraft- und Druckmessung Einsatz finden können, die sich bereits auf anderen Gebieten wie Materialuntersuchungen im Metall- oder Kunststoffbereich bewährt haben.

Es kann vorteilhaft sein, an einem Teigstück mehrere Festigkeitsmessungen durchzuführen, weil beispielsweise der Teigzustand im mittleren Bereich anders als im Randbereich eines Teigstücks sein kann. Die mehreren Messungen insbesondere pro Teigstück lassen sich zweckmäßig zu einem Meßzyklus zusammenfassen, für den eine Ablaufsteuerung speziell programmiert sein kann, um beispielsweise am Teigband oder Teigstück mehrere Festigkeitsmessungen zeitlich hintereinander und/oder lokal an unterschiedlichen Stellen durchzuführen.

Gemäß einer konkreten Ausgestaltung des erfindungsgemäßen Meßverfahrens wird in Wirkungsverbindung mit dem Sensor eine zunehmende Auslenkung von Teigmasse herbeigeführt und dabei über den Sensorausgang in der Steuerung der Verlauf der Kraft oder des Drucks in Abhängigkeit vom Auslenkungsweg oder umgekehrt der Auslenkungsweg in Abhängigkeit vom Verlauf der Kraft oder des Drucks aufgezeichnet, gespeichert und zu Steuerungs- oder Regelungszwecken ausgewertet.

Um einen hohen Teigdurchsatz und eine hohe Produktionsgeschwindigkeit bei ausreichend zuverlässiger Erfassung des Gesamt-Teigzustandes zu ermöglichen, werden gemäß einer vorteilhaften Ausgestaltung mehrere Sensoren gleichzeitig auf die gegebenenfalls getaktete Teigfördergeschwindigkeit synchronisiert und dabei an lokal unterschiedlichen Stellen des Teigbands oder des Teigstücks mit der Teigmasse zur Festigkeitsmessung in Kontakt gebracht.

Bei an sich bekannten, maschinellen Teigformlinien erfolgt die Formung von Teigböden oder -bändern nach zwei grundlegenden Methoden. Der Teig wird entweder geteilt und danach in einer Form rundgewirkt ("ball and sheet system"), oder die Teiglinge werde am Ende eines Ausrollprozesses aus einem kontinuierlichen Teigband herausgeschnitten. Bei jeder Teigformtechnik bildet die Linie aus hintereinander angeordneten Teigverarbeitungsmaschinen ein komplex wirkendes Maschinensystem zur Volumendosierung mit jeweils spezifischer Wirkung der Einzelelemente, wobei die Teigformlinie nach dem Kneter und vor dem Backofen eingebunden ist. Es ist bekannt, dass die Reihenschaltung der Einzelelemente in der Teigformlinie eine jeweils spezielle Teigformung durch Aufbringen eines Deformationszustandes und damit die Einleitung eines Fließvorgangs bewirkt.

Die bekannte Technik der maschinellen Teigformlinien mit einzelnen Teigverarbeitungsgerätschaften und -maschinen benötigt aber Teig ausreichend guter Qualität, mit denen gewünschte Durchsätze sicher gefahren werden können.

Es ist bekannt, dass nach dem Knetvorgang der Teig nach etwa 20-minütiger Bandgare bei Einsatz von kontinuierlichen Knetsystemen in einen Vorportionierer gelangt. Der Teig wird hier in definierten Teigstücken portioniert. Danach wird aus den Teigstücken im Teigbandformsystem ein kontinuierliches Teigband geformt. Insbesondere kann dabei die Teigformlinie mit einem Satellitenkopfsystem beginnen. Bei bekannten 3-Walzen-Systemen wird der Teig auf eine Walze gepresst und wesentlich stärker belastet. Die Kombination aus Satellitenkopf und Transportband ermöglicht dagegen einen schonenderen Roll- und Formprozess. Das Ziel des nachfolgenden Ausrollprozesses ist das schonende Reduzieren des Teigbandes auf eine gewünschte Teigendstärke. Sie beträgt bei Tiefkühlpizza etwa 3-5 Millimeter. Hierzu werden kombiniert Multiroller- bzw. Satellitenkopfsysteme (Anzahl beispielweise 2), ein Querwalzwerk mit Rechtslaufrollern/Linkslaufrollern und Schlichtwerke (Kalibrierköpfe bspw. in der Anzahl 2) eingesetzt. Moderne Satellitenkopfsysteme reduzieren besonders dicke Teigbänder sehr schonend durch eine gleichzeitige Roll- und Stoßbewegung. Der Teig wird dabei jeweils zwischen den einzelnen Rollen-Stößen kurz entspannt. Das Protein-Netzwerk des Teiges wird dadurch nur geringfügig belastet. Durch den Einsatz von Querwalzwerken werden die Teigbänder auf Produktionsbreite verbreitert. Die Längsspannungen in der Kleberstruktur sollen dabei egalisiert werden. Die nachfolgenden Kalibrierkopfeinheiten reduzieren die Teigbänder auf die erforderliche Endstärke. Zudem sorgen sie für eine hohe Oberflächen- und Gewichtsgenauigkeit der Teige. Bevor schließlich die Teiglinge aus dem Teigband herausgestanzt werden, ist noch eine bspielsweise 10-minütige Entspannungsgare zur Verminderung von Längsspannungen zweckmäßig, um eine qualitativ hochwertige Porenstruktur zu entwickeln.

Allerdings können schon am Display bekannter Knetsysteme bei der Dosage der Ingredienzien zeitliche Differenzen im Dosiervorgang erkannt werden. Wie visuell und mit kinästhetischen Methoden feststellbar, ändert sich im Verlauf eines Produktionstages merklich die Konsistenz der eingesetzten Teige. Insbesondere die Verarbeitung eines weichen Teigs führt zu größeren Problemen bei der Massenportionierung. Die nicht unbedeutenden Schwankungen in der Teigqualität während eines Produktionstages sind Ursache eines erheblichen Bedienungsaufwandes und bedingen Unterschiede in der sensorischen Qualität der Teigware des Endproduktes. Beim direkten Vergleich von in die Teigformlinie eingehenden Teigen können sich der Elastizitätsmodul und die maximale Teigdehnungskraft um mehr als die Hälfte unterscheiden. Diese über einen Produktionstag auftretenden, erheblichen Teig-Qualitätsunterschiede bilden Störgrößen für die Teigverarbeitungslinie. Insbesondere durch ein Querwalzwerk werden aufgrund der höheren Fluidität die Breiten der Teigbahnen zu sehr ausgeformt. Überbreiten müssen durch Beschneidung entfernt werden, damit im Gärschrank nicht Teigüberhänge auftreten. Zur Abhilfe werden durch das Bedienpersonal Korrekturen an Maschineneinstellungen oder auch an der Qualität des eingehenden Teiges selbst eingeleitet. Die Vornahme solcher Änderungen und Korrekturen können eine Stunde und mehr in Anspruch nehmen.

Der Erfindung liegt weiter die Aufgabe zugrunde, beim Teiggären und/oder Teigformen/-aufbereiten eine gleichmäßige Qualität entsprechend einer vorherigen Spezifikation des jeweiligen Prozess-Endproduktes zu gewährleisten.

Zur Lösung wird das im Patentanspruch 10 angegebene Regelungs- und Steuerungsverfahren für einen Teigformungs-/-aufbereitungsprozeß mit mehreren Subprozessen vorgeschlagen. Vorteilhafte Ausgestaltungen und Einzelmerkmale ergeben sich aus den davon abhängigen Ansprüchen.

Erfindungsgemäß werden also die objektiven Materialeigenschaften des Teiges während des Formprozesses in der Teigformlinie erfaßt und als Regelgrößen genutzt. Dies kann nach einer vorteilhaften Ausgestaltung bereits unmittelbar nach Austritt des Teiges aus dem Kneter und bei Zuführung auf die Teigformlinie erfolgen. Insbesondere lassen sich der Einfluß der Teigkonsistenz sowie deren viskoelastisches Verhalten während der Verarbeitung in der Teigformlinie ermitteln und bewerten. Indikatoren sind der Elastizitätsmodul beziehungsweise die Dehnung als Maß für die durch Energiezufuhr bewirkte Deformation und als Funktion der auf den Teig einwirkenden Schergeschwindigkeiten. Das Verformungsverhalten als Folge der Wirkpaarung Material (Teig) und Maschinentechnik in Form der nacheinander geschalteten Bearbeitungsstufen der Teigformlinie wird erfindungsgemäß sensorisch erfaßt und zur Verstellung bzw. Nachkorrektur der Teigbearbeitungsorgane regelungstechnisch rückgeführt. In diesem Zusammenhang ist es vorteilhaft, das Teig-Verhalten anhand seiner elastischen Komponente bzw. seines Elastizitätsmoduls in Echtzeit zu erfassen. Die Elastizität stellt die geeignetste Meßgröße zur Bewertung des Einflusses der Maschinenwirkung auf die Strukturstabilität des Teiges dar.

Gemäß einer vorteilhaften Ausgestaltung des erfindungsgemäßen Verfahrens werden am Teig Dehnungsmessungen in Echtzeit durchgeführt, woraus computergestützt das viskoelastische Teigverhalten ermittelt und mit zugeordneten Führungsgrößen zur Gewinnung entsprechender Stellbefehle für die Teigbeeinflussung verglichen wird. Dazu eignen sich für den Online-Produktionsbetrieb geeignete Dehnungsmeßgeräte, für die auf an sich bekannte Dehnungsmeßprinzipien zurückgegriffen werden kann. Der zu verarbeitende Teig wird dabei gewissen mechanischen Belastungen unterzogen.

Andererseits ist es im Rahmen der Erfindung auch denkbar, das Elastizitätsmodul über die Weiterverarbeitung von rückgeführten Meßsignalen zu gewinnen, die ohne Berührung des Teigs abgeleitet sind. Die berührungslose Echtzeiterfassung von Teigmaterialparametern läßt sich dazu auf der Basis opto-elektronischer Verfahren durchführen.

Nach einer weiteren vorteilhaften Ausbildung werden in Echtzeit in der Teigumgebung Luftfeuchtigkeitsmessungen und/oder am Teig Dicke- und/oder Temperaturmessungen vorgenommen. Diese bilden Istwerte zum Vergleich mit Sollwerten für eine oder mehrere Subprozeß-Regelungen.

Im Rahmen der Erfindung liegt es, die Verarbeitungseigenschaften des Teiges vor allem schon bei Aufgabe auf die Teigverarbeitungslinie objektiv zu erfassen, Änderungen der Teigqualität zu erkennen und dies den Maschinenkomponenten der Teigverarbeitungslinie zu deren Steuerung und/oder Regelung zu signalisieren. Dadurch läßt sich eine stabile Linienfahrweise erzielen, welche einen wesentlichen Beitrag zum Qualitätssicherungssystem und -management für die Teigverarbeitung liefern kann.

Auf der Basis der Erfindung lassen sich für die Abläufe der Teigaufbereitung neue, prozessbegleitende Verfahren zur Echtzeit-Erfassung der Teigqualität bezüglich des Verarbeitungsverhaltens entwickeln. Insbesondere wird mit der Erfindung die Möglichkeit eröffnet, ein Prozessleitsystem mit nachgeschalteten Regelkreisen für die maschinelle Teigformlinie und die "Textur" zu erarbeiten. Ferner ist die Möglichkeit geschaffen, die Ausbeute und den Wirkungsgrad der maschinellen Teigformlinie zu erhöhen. Ferner ist die Perspektive eröffnet, Mehle ohne Zusätze von Backmischungen (wodurch ein Schutz vor Allergieauslösungen erzielbar ist) unter Erzielung einer gleichmäßigen, gleichbleibenden Teigqualität zu verarbeiten, und zwar durch die erfindungsgemäß auf der Basis von globaler Regelungstechnik objektivierte Fahr- beziehungsweise Betriebsweise der Teigformlinie nebst dem Prozeß der Teiggärung. Auf der Basis der Erfindung kann ein automatisiertes Qualitätsmanagement realisiert werden.

Im Rahmen der Erfindung liegen auch Mikrozugversuche zur Bestimmung der Festigkeit und Dehnbarkeit von Teig und Kleber während des Teigformprozesses zur Bewertung der in der Gärphase ablaufenden Strukturveränderungen. Zur Charakterisierung von Weizenteig und -kleber werden entsprechende Stränge mit einer Textur-Prüfmaschine so lange gedehnt, bis sie reißen. Die erhaltenen Kraft-Weg-Diagramme sind sortenspezifisch und geben Aufschluß über die Wirkung von Zusätzen oder technologischen Operationen. Versuchsvoraussetzung ist eine standardisierte Breite der Teigproben, beispielsweise 10 Millimeter. Dies kann auch manuell mit einem Schneidrad auf einem Transportblech erzeugt werden. Die Teigprobe wird prinzipiell in Bandlaufrichtung gewonnen. Die Auswertemethodik ist wie folgt: Eine Messkurve, bei der die aufzuwendende Dehnungskraft über die Dehnung in Millimeter aufgetragen ist, weist zumindest begrenzt einen linearen Abschnitt auf. Da der Elastizitätsmodul nur für lineare Messkurvenläufe definiert ist, wird abhängig vom Probenentnahmeort ein linearer Bereich abgegrenzt. Innerhalb dieses Bereiches haben die Messkurven eine nahezu linearen Verlauf.

Die sichere und zuverlässige sowie qualitätsorientierte Arbeitsweise einer Teigformanlage (Teiglinie) ist abhängig von der Einhaltung verbindlicher materialwissenschaftlicher Parameter des Teiges. Als Kriterium der der Anlage zugeführten Teigkonsistenz kann im Rahmen der Erfindung der Youngsche Elastizitätsmodul dienen. Durch den Betreiber der maschinellen Teigformungslinie ist die Teigqualität insbesondere anhand dieses Moduls mit einer möglichen Toleranzspanne zu definieren. Liegt der Teig außerhalb dieser verbindlichen Qualitätsnormen vor, kann seitens des Linienherstellers keine sichere Bearbeitung im vereinbarten Massentoleranzbereich von +/- 4,5 % garantiert werden.

Eine besonders vorteilhafte Ausgestaltung der Erfindung besteht darin, die von der Teigbildungsanlage (Kneter/Mischer) zur Teigformungslinie gelangenden Teig-Konsistenzen kontinuierlich zu erfassen, um das Qualitätsmanagement und insbesondere das HACCP-Konzept bei etwaigen Fehlbedingungen (falscher Mehlqualität) zu sichern. Dazu dient folgende besondere Ausbildung der erfindungsgemäßen Verfahrensweise: Im ersten Schritt wird der im 15-Minuten-Takt zur Teigformungslinie gelangende Teig untersucht und in Form einer Tageskurve beschrieben. Diese Tageskurve kann zur Definition von Abweichungen im Linienbetrieb genutzt werden. So kann der Betrieb der Teigformungsanlage bzw. -linie vom Eingang einer definierten Teigqualität mit optimalen Verarbeitungs- bzw. Textureigenschaften abhängig gemacht werden. Dazu ist die Messung der Teigfestigkeit und die resultierende Ableitung des Elastizitätsmoduls zur Realisierung der Rückkopplung für die Regelkreise oder die Mehrgrößenregelung zweckmäßig. Konsistenzänderungen lassen sich so als mögliche Regelgröße anzeigen.

Zur Durchführung des vorstehenden Meßverfahrens in Verbindung mit der dazu genannten Erfindungsaufgabe wird im Rahmen der allgemeinen erfinderischen Idee auch die im Patentanspruch 16 angegebene Meßanordnung vorgeschlagen. Vorteilhafte Ausgestaltungen und Einzelmerkmale ergeben sich aus den abhängigen Ansprüchen.

Demnach ist der Sensor von einem motorisch angetriebenen Stellorgan erfaßt und verstellbar. Letzteres ist geeignet, von der Steuerung kontrolliert und in dem Zeitpunkt angesteuert zu werden, in welchem sich für den Sensor eine geeignete Meßstellung gegenüber dem zu messenden Teig ergeben hat. Der Sensor läßt sich dann durch das Stellorgan kontaktgebend zum Teig hin oder auch von diesen weg verstellen. Wie bereits weiter oben angesprochen, ist ein derartiges, aus Sensor und Stellorgan gebildetes Gesamt-Meßgerät in dem Bereich Metall- und Kunststoff-Materialprüfung an sich bekannt, wenn auch ohne jegliche Anpassung zum Einsatz in einer automatische Teigformlinie. Dazu sind bei der erfindungsgemäßen Meßanordnung Kopplungsmittel vorgesehen, die ebenfalls von der Steuerung kontrolliert oder kontrollierbar sind und der kinematischen Synchronisation des Standorts bzw. der Stellung des Meßgeräts gegenüber dem zu Produktionszwecken geförderten Teig dienen. Indem die Kopplungsmittel und das Meßgerät miteinander in mechanisch starrer Verbindung stehen, können die Kopplungsmittel bei entsprechender Ansteuerung dafür sorgen, dass gemäß einer Merkmalsalternative das Meßgerät mit dem geförderten Teig bzw. der Teig-Fördereinrichtung mitbewegt wird. Mithin läßt sich die baulich-konstruktive Anpassung des Meßgeräts an den Prozeß der automatischen Teigbearbeitung mit nur einer baulichen Maßnahme, dem Einführen der Kopplungsmittel, bewerkstelligen, welche konstruktiv und funktionstechnisch relativ einfach ausgeführt sein können.

Dazu ist gemäß einer vorteilhaften Ausführungsvariante die Realisierung der Kopplungsmittel mit einer Parallelführung vorgesehen, die parallel zur Teig-Fördereinrichtung verläuft. In oder längs der Parallelführung läßt sich ein Trägerwagen führen und mit der Fördereinrichtung und dem geförderten Teig mitbewegen. Der Trägerwagen kann gleichsam ein Transportmittel für das Meßgerät bilden und dieses entsprechend der Teigförderung mitbewegen und auch wieder in eine Ausgangsstellung zurückbewegen.

Um beim Teigstück oder Teigband den Zustand möglichst breitflächig erfassen zu können, insbesondere sowohl in den mittleren als auch in den Randbereichen, ist gemäß einer vorteilhaften Ausgestaltung auch eine Querverstellführung für das Meßgerät vorgesehen. Diese kann zweckmäßig mit einem eigenen Antriebsmotor versehen sein, der von der (Meß-) Steuerung kontrolliert oder kontrollierbar ist. Je nach Meßprogramm können verschiedene, anzufahrende Punkte beim Teigstück oder Teigband eingestellt sein. So läßt sich für den Sensor ein umfassender und komplexer Meßzyklus mit mehreren Teigfestigkeitsmessungen an unterschiedlichen Teigstellen gegebenenfalls zu unterschiedlichen Zeitpunkten programmieren. Der Meßzyklus kann wiederholt beispielsweise zu äquidistanten Zeitpunkten durchgeführt werden.

Zur präzisieren Synchronisation mit den Bewegungs- und gegebenenfalls Stillstandszeiten des geförderten Teigs sowie zur Erhöhung der Meßgenauigkeit ist gemäß einer vorteilhaften Ausbildung vorgesehen, dass die Kopplungsmittel eine Basisplatte umfassen, die dem Sensor gegenüberliegend angeordnet ist bzw. gehalten wird. Dies kann beispielsweise über den Trägerwagen an der Parallelführung erfolgen. Damit ist die Möglichkeit eröffnet, dass die Fördereinrichtung, beispielsweise ein Teigförderband, mit gegebenenfalls darauf aufliegendem Teig (strangförmiges Band oder diskrete Einzelstücke), zwischen der unteren Basisplatte und dem oberen Sensor gelangen. Diese Stellung ist günstig, um den Sensor in Kontakt mit dem Teig zu bringen. Dessen Lage wird dabei durch die Basisplatte für die Festigkeitsmesung stabilisiert beziehungsweise präzise gehalten.

Die Mitbewegung des Meßgeräts mit der Teig-Förderbewegung läßt sich gemäß einer Ausführungsvariante dadurch bewerkstelligen, dass die Kopplungsmittel ein oder mehrere Mitnehmerelemente aufweisen, die von der Steuerung betätigbar ausgebildet und mit der Fördereinrichtung verbindbar sind. Zweckmäßig sind die Mitnehmerelemente mit den Kopplungsmitteln dadurch bewegungsübertragend verbunden, dass sie am Trägerwagen fest angebracht sind.

Damit Meßzyklen mit jeweils gleichem Ablauf wiederholt werden können, muß der Druck- oder Kraftsensor wiederholt in eine vorbestimmte Ausgangsstellung gebracht werden. Dem dient eine Ausgestaltung der erfindungsgemäßen Meßanordnung, nach welcher die Kopplungsmittel einen ortsfest abgestützten und am Meßgerät, gegebenenfalls am Trägerwagen oder an der Basisplatte angreifenden sowie von der Steuerung kontrollierbaren Rückstellantrieb aufweisen. Dieser ist so ausgebildet, dass er zur Verstellung des Meßgeräts entgegen der Förderrichtung betätigbar, beispielsweise in Gegenförderrichtung teleskopartig ausfahrbar ist.

Zur gerätetechnischen Realisierung des zuvor genannten Teigform-Regelungsund Steuerungsverfahrens und im Zusammenhang mit der dazu genannten Erfindungsaufgabe wird die im Patentanspruch 25 angegebene Regelungs- und Steuerungsanordnung mindestens für den Prozeß des Teigformens/-aufbereitens vorgeschlagen. Vorteilhafte Ausgestaltungen und Einzelmerkmale ergeben sich aus den abhängigen Ansprüchen.

Also wird erfindungsgemäß zumindest beim Teigformen/-aufbereiten bei einem oder mehreren der Subprozesse die Meßanordnung zur Ermittlung der Teigfestigkeit eingesetzt. Die Meßanordnung ist derart angeordnet und ausgebildet, dass sie sich kinematisch mit den Förderungsbewegungen des Teiges bei einem oder mehreren der Subprozesse koppeln läßt. Dazu eignen sich besonders die oben genannten Kopplungsmittel der erfindungsgemäßen Meßanordnung. Damit wird der Vorteil erzielt, dass Teigfestigkeitsmessungen zur Beobachtung der Teigqualität in regelmäßigen, insbesondere äquidistanten Zeitabständen durchgeführt werden können, ohne dass der Teigdurchsatz und die Produktionsausbeute an Teiglingen beeinträchtigt werden.

Gemäß einer weiteren Erfindungsausbildung ist die vorzugsweise digitalelektronisch oder rechnergestützt realisierte Regeleinrichtung so eingerichtet oder programmiert, dass darin die Sensorsignale der Festigkeitsmessung zum Elastizitätsmodul und gegebenenfalls dessen funktionellen Verlauf weiterverarbeitet werden können. Danach läßt sich ein Soll-/lstwertvergleich mit vorgegebenen Toleranzgrenzen für die Teigelastizität durchführen. Damit eröffnet sich vorteilhaft der weitere Weg, über die Steuerungsund Regeleinrichtung so auf die Stellglieder für die Subprozesse zur Teigformung einzuwirken, dass ein spezifiziertes Toleranzband für den Teig-Elastizitätsmodul auch am Ende der Teigformungslinie eingehalten wird.

Im Rahmen der Erfindung wird bei einer Regelungs- und/oder Steuerungsanordnung zur Teigbearbeitung vorgeschlagen, dass die Stellglieder wenigstens zweier Prozesse oder Subprozesse zu ihrer Ansteuerung oder Einstellung mit einer gemeinsamen Steuerungs- oder Regeleinrichtung verbunden sind, welche eingangsseitig mit einer Prozeßleitsteuerung gekoppelt ist, die zur Erzeugung und Ausgabe von Führungsgrößen an die Steuerungs- oder Regeleinrichtung koordiniert und/oder synchronisiert für zwei oder mehr Prozesse eingerichtet und/oder programmiert ist. Dabei kann in Weiterführung dieses Erfindungsgedankens die Regeleinrichtung mit wenigstens zwei der Prozesse oder Subprozesse, (welche dabei Regelstrecken bilden) über eine jeweils zugeordnete Istwert-Sensorik für Teigeigenschaften (insbesondere Elastizitätsmodul) gekoppelt werden. Ferner ist die Regeleinrichtung zweckmäßig mit jeweils einem Prozeß zugeordneten Soll-/lstwert-Vergleichsgliedern versehen, die je mit einer der Istwert-Sensoriken verbunden sind.

Auch ist mit der Erfindung die Basis geschaffen, im Rahmen einer vorteilhaften Ausbildung zwischen wenigstens einem ersten und einem zweiten Teigbearbeitunsprozeß (z. B. Teigbildung oder Teigformung) eine von der Regeloder Steuerungseinrichtung über ein eigens zugeordnetes Stellglied kontrollierbare Teig-Fördereinrichtungen anzuordnen. Dies kann auch zwischen zwei der genannten Subprozesse erfolgen. In der Regel- oder Steuerungseinrichtung ist ein derart programm- und/oder schaltungstechnisch ausgebildetes Modul vorgesehen, dass abhängig von am Ende des ersten Prozesses erfaßten Teig-Istwerten die Teig zum zweiten Prozeß bewegende Fördereinrichtung aktivierbar beziehungsweise betätigbar ist. Damit ist die vorteilhafte Möglichkeit geschaffen, eine etwaige mangelhafte Qualität von Teigmasse beispielsweise aus einem Knet- oder Mischungsprozeß (erster Prozeß, Teigbildung) über die Istwert-Sensorik rechtzeitig zu erkennen und durch Abschalten der Fördereinrichtung zu verhindern, dass dieser Teig mangelhafter Qualität in eine nachgeschaltete, maschinelle Teigformlinie gelangt (zweiter Prozeß, Teigformung).

Im Rahmen der allgemeinen erfinderischen Idee liegt es auch, die genannte Regelungs- und Steuerungsanordnung auf einen einzigen der genannten Prozesse anzuwenden. So kann der (Haupt-)Prozeß "Teig bilden" Subprozesse wie Dosieren von Teiggrundstoffen und -ingredienzien, Kneten beziehungsweise Mischen und der (Haupt-)Prozeß "Teig formen/-aufbereiten" Subprozesse wie Vorformen, Kalibrieren, Ausrollen, Querwalzen, Schneiden in einzelne Teigstücke, Wickeln, Biegen und/oder Ineinanderschlingen einzelner Teigstücke umfassen. Diese ganzen Subprozesse eines Hauptprozesses lassen sich mit der aufgezeigten Regelungsstruktur vorteilhaft aufeinander synchronisieren und/oder koordinieren, wenn erfindungsgemäß zwei oder mehr diese Subprozesse bewirkende oder beeinflussende Stellglieder zu ihrer Ansteuerung oder Einstellung mit einer gemeinsamen Steuerungs- oder Regeleinrichtung verbunden sind, welche eingangsseitig mit einer Prozeßleitsteuerung verbunden ist. Diese ist zur Erzielung und Ausgabe von Führungsgrößen an die Steuerungs- oder Regeleinrichtung in für zwei oder mehr Subprozesse koordinierter und/oder synchronisierter Weise eingerichtet und/oder programmiert.

Weitere Einzelheiten, Merkmale, Vorteile und Wirkungen auf der Basis der Erfindung ergeben sich aus der nachfolgenden Beschreibung bevorzugter Ausführungsformen der Erfindung sowie aus den Zeichnungen. Diese zeigen in:
- Figur 1: in vereinfachter perspektivischer Darstellung ein Beispiel für eine erfindungsgemäße Meßanordnung,
- Figur 2: einen vergrößerten Ausschnitt aus Figur 1,
- Figur 3: in einer vergrößerten Vorderansicht den mit einer Druckplatte in ein Teigband auf einem Teig-Förderband eindringenden Kraft- oder Drucksensor,
- Figur 4: ein Kraft-Weg-Diagramm entsprechend dem in Figur 3 dargestellten Meßvorgang,
- Figur 5: in schematischer Blockdarstellung ein globales Regelungssystem mit Prozeßleitsteuerung für eine Mehrzahl von Ist- und Stellwerten, und
- Figur 6: die umfassende Regelung und Steuerung der Subprozesse des Hauptprozesses "Teigformen/-aufbereiten" am Beispiel einer maschinellen Formlinie für Brezelteiglinge.

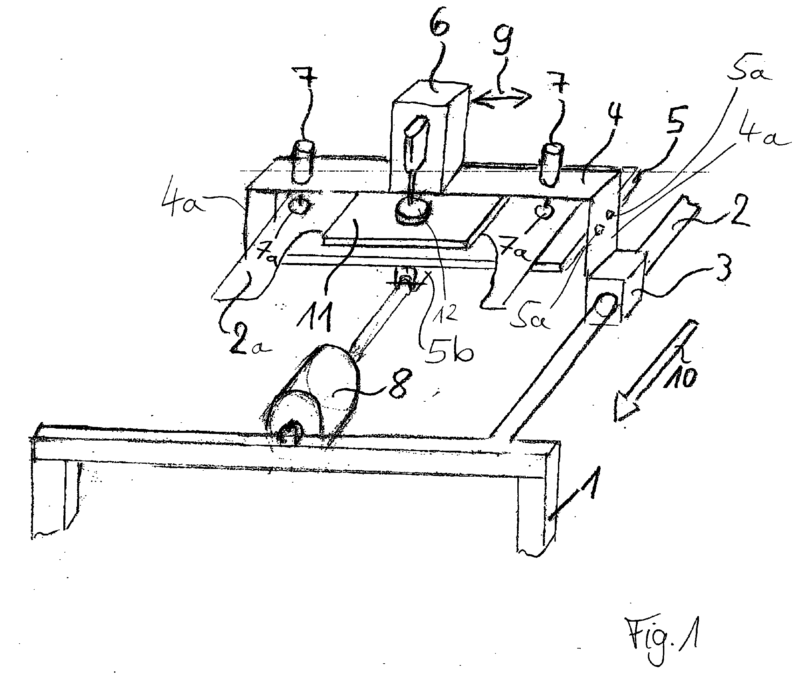

Gemäß Figur 1 weist die erfindungsgemäße Meßanordnung ein Gestell 1 auf, von dem ein oder mehrere Führungsstangen 2 parallel zu einer Transport- oder Förderrichtung 10 eines Teig-Förderbandes 2a vorspringen. Jede Führungsstange 2 ist von einer Führungshülse 3 umgeben, welche fester Bestandteil eines Trägerwagens 4 ist. Diesem ist eine das Förderband 2a quer überbrückende Gestalt verliehen. An zu je einer Längsseite des Förderbandes 2a ausgebildeten Vertikalwangen 4a d es Trägerwagens 4 ist mit ihren anliegenden Schmalseiten eine Basisplatte 5 mit Verschraubungen 5a so befestigt, dass sie die beiden Vertikalwangen 4a unterhalb des Förderbandes 2a verbindet. Sie kann so eine Auf- oder Unterlage für das Förderband 2a mit darauf liegendem Teig 11 gegenüber einem sich in den Teig eindrückenden Druckplattensensor 12 eines Meßgeräts 6 für Teigfestigkeit bilden. Dieses arbeitet nach einem Meßprinzip, wie es bei handelsüblichen Meßgeräten für Materialuntersuchungen im Metall- oder Kunststoffbereich bekannt ist.

Gemäß Figur 1 ist das Meßgerät 6 auf der Oberseite des das Förderband 2a überbrückenden Querstegs des Trägerwagens 4 angebracht, und zwar vorzugsweise über eine Querverstellführung 9 von einem Rand zum gegenüberliegenden Rand des Förderbandes 2a hin und her verstellbar. Die (zeichnerisch nur angedeutete) Querverstellführung 9 kann mit einem elektronisch steuerbaren Antriebsmotor versehen sein, so dass ein (nicht gezeichneter) Steuerungsrechner mit einem Meßprogramm eine Verschiebung und Einstellung des Standorts des Meßgeräts 6 relativ zum Förderband 2a und zum darauf liegenden Teig 11 bewirken kann.

Ferner wirkt der Steuerungsrechner zweckmäßig auf einen Rückstellantrieb 8 ein, der gemäß Figur 1 beispielsweise mit einem teleskopartig ausfahrbaren Pneumatikzylinder realisiert sein kann. Am einen Ende ist er an dem ortsfesten Gestell 1 abgestützt. Am anderen Ende greift der Rückstellantrieb 8 an der Basisplatte 5 an, wozu er von einem U-artigen Befestigungselement 5b an der Unterseite der Basisplatte 5 umfaßt ist.

Gemäß Figur 1 ist der Trägerwagen 4 ferner mit Mitnehmerelementen 7 versehen, die jeweils an einem der beiden Endbereiche des Trägerwagen-Querstegs angeordnet sind, so dass sie zu beiden Seiten des Teigs 11 in den Randbereichen des Förderbandes 2a angreifen können. Gemäß gezeichnetem Beispiel sind die Mitnehmerelemente 7 ebenfalls mit Pneumatikzylinder realisiert, die bei entsprechender Ansteuerung durch den Steuerungsrechner teleskopartig ausfahrbar sind. Zweckmäßig ist eine Anordnung derart, dass sie dabei den Trägerwagen-Quersteg von dessen Oberseite her durchsetzen sowie darauf abgestützt sind und mit ihren Klemmplatten 7a am Ende das Förderband 2a gegen die Oberseite der mit dem Trägerwagen 4 fest verbundenen Basisplatte 5 drücken können. Dadurch kommt es bei Förderbewegung 10 des Förderbandes 2a zu einer Mitnahme des Trägerwagens 4 entlang der Führungsstangen 2. Der Rückstellantrieb 8 ist dabei kraftlos zur Ausübung eines Freilaufs einzustellen.

Zur Funktionsweise dieser erfindungsgemäßen Meßanordnung wird noch folgendes ausgeführt:

Gemäß Programm des Steuerungsrechners wird eine vorgegebene Position des Meßgerätes 6 auf dem Trägerwagen 4 eingestellt. Entsprechend einem vorprogrammierten Meßzyklus werden am Teig 11 (beispielsweise Teigband) ein oder mehrere Messungen in bestimmten Zeitabständen und lokalen Bereichen des Teigbandes durchgeführt. Ein solcher Meßzyklus kann nach beliebigen Zeitabständen wiederholt werden.

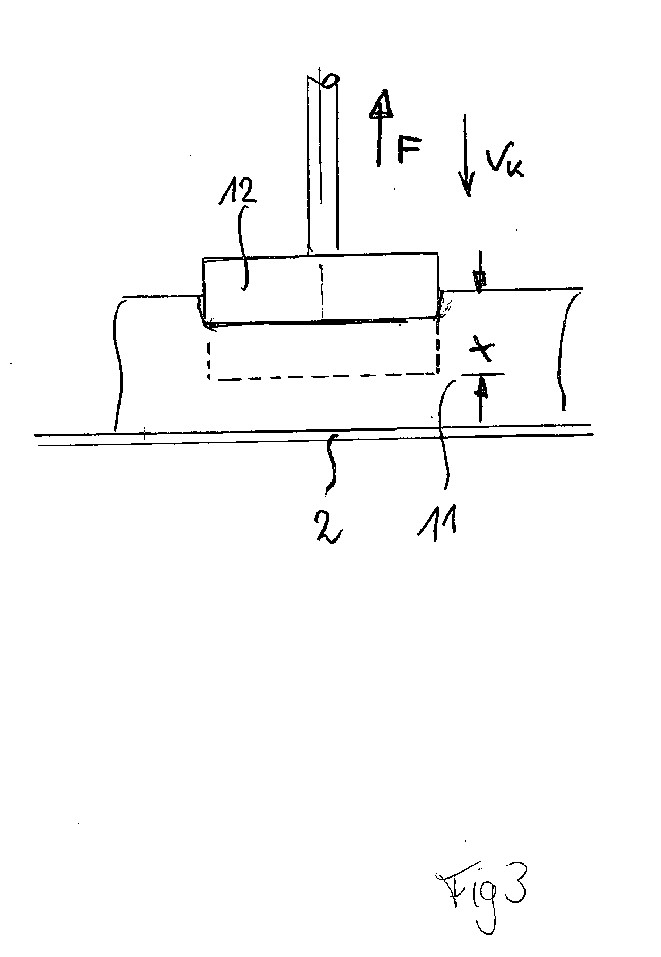

Zu Beginn einer Teigfestigkeitsmessung ist der Trägerwagen 4 mit der Förderbandgeschwindigkeit zu synchronisieren. Dazu werden die pneumatischen Mitnehmerzylinder 7 ausgefahren, um das Förderband 2a auf die Oberseite der Basisplatte 5 zu klemmen. Dadurch wird erreicht, dass das Meßgerät 6 auf dem Trägerwagen 4 mit dem Förderband 2a entlang der Führungsstange 2 mitbewegt wird. Sobald diese Förderbewegung einsetzt, wird der Meßvorgang gestartet: Der Druckplattensensor 12 wird auf die Oberfläche des Teigs 11 zubewegt. Der Steuerungsrechner empfängt Meßsignale aus dem Meßgerät und zeichnet einen Meßwerteverlauf auf, sobald der Druckplattensensor 12 die Teigoberfläche berührt und in den Teig 11 eindringt (Figur 3). Dabei wird ein Druckmeßumformer, der mit dem Druckplattensensor in Wirkungsverbindung steht, betätigt. Druckmeßumformer sind an sich in großer Auswahl bekannt. Dessen elektrische Meß-Ausgangssignale werden von der Rechnersteuerung in der genannten Weise verarbeitet.

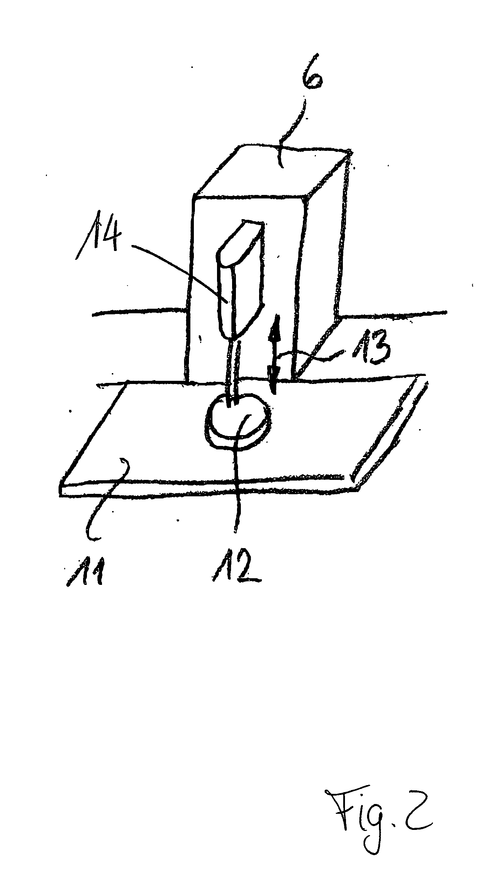

Gemäß Figur 2 wird das Zubewegen des Druckplattensensors 12 auf den Teig 11 und das nachfolgende Entfernen durch eine vertikale Hubbewegung 13 erreicht, die mittels eines vom Steuerungsrechner kontrollierbaren Stellorgans 14 entsprechend dem programmierten Meßzyklus ablaufen sein kann. Das Stellorgan 14 kann mit einem vorzugsweise linearen Antriebsmotor in einem Ansatz des Gehäuses des Meßgerätes 6 untergebracht sein.

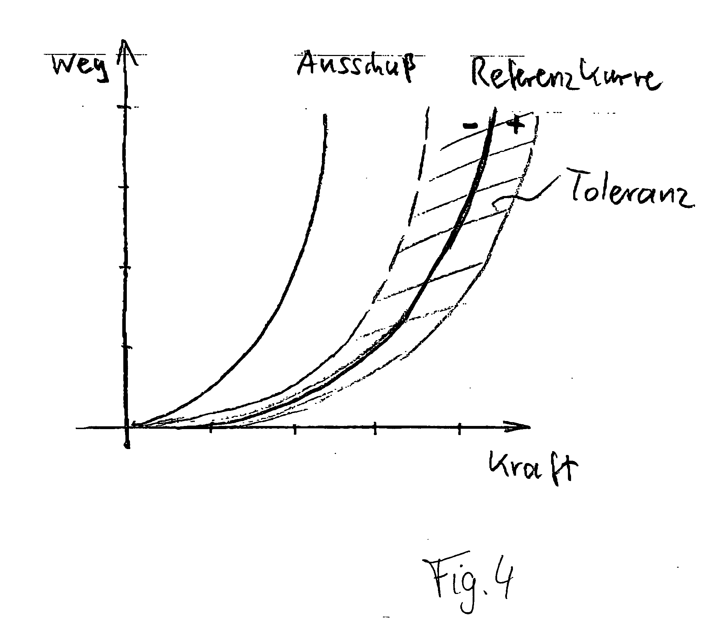

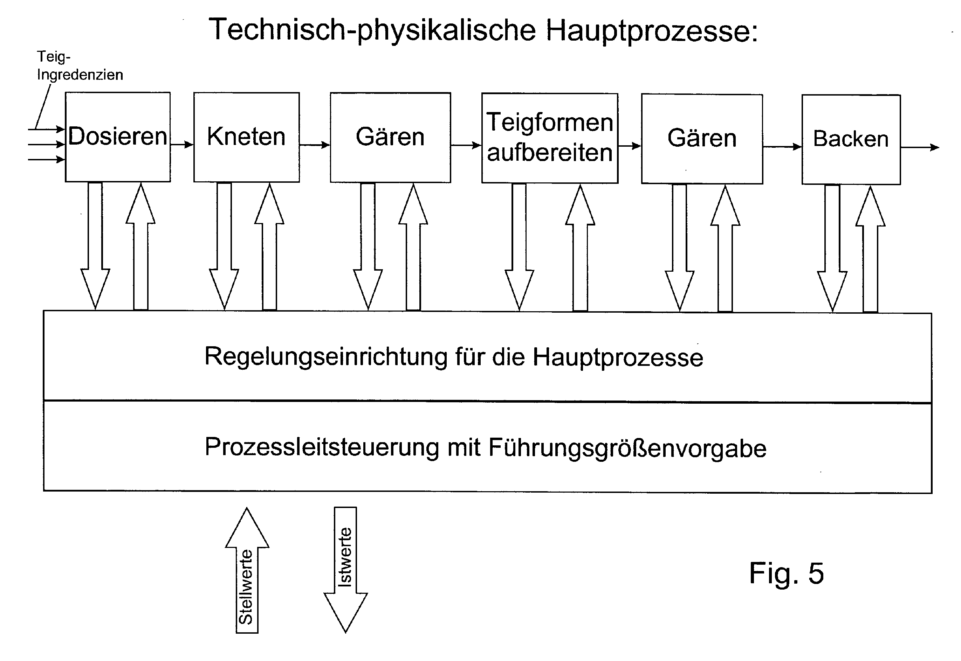

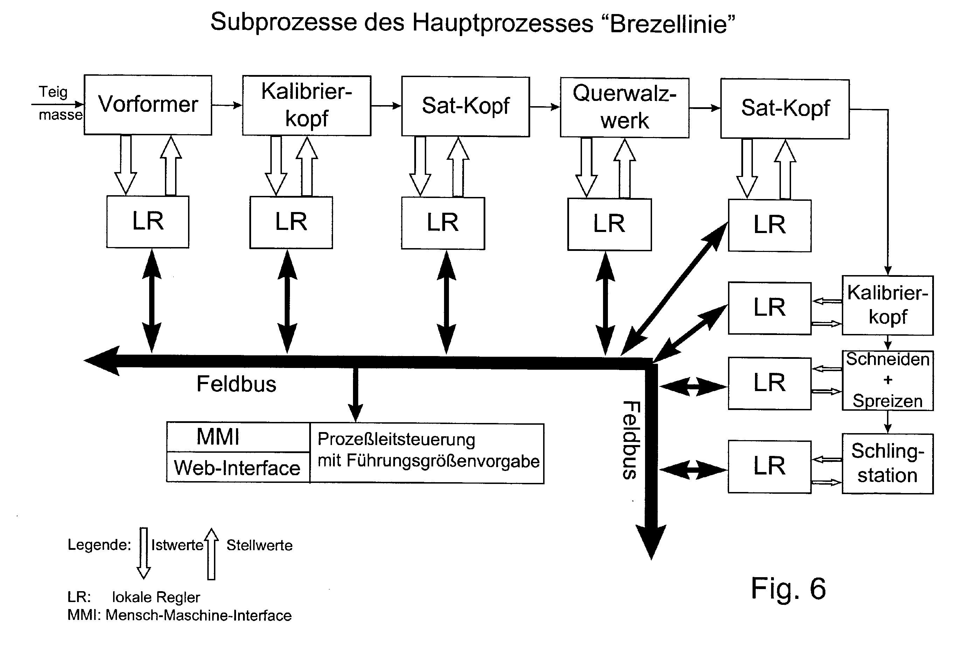

Mittels des Stellorgans 14 läßt sich nach Figur 3 der Druckplattensensor 12 mit konstanter Geschwindigkeit Vk in den Teig 11 mit einer definierten Eintauchtiefe X eindrücken. Die Widerstandskraft F des Teiges gegen das Eindringen des Druckplattensensors 12 ist ein Maß für die Festigkeit und Qualität des Teiges und läßt sich mit dem integrierten Druckmeßumformer erfassen und im Steuerungsrechner aufzeichnen. Dazu ist der Meßbereich und ein entsprechendes Meßprogramm unter rheologischen Gesichtspunkten bezüglich Teigeigenschaften erstellt und im Steuerungsrechner implementiert. Dieser kann im Zuge des Meßprozesses ein Kraft-Weg-Diagramm gemäß Figur 4 intern erzeugen, das mittels weiterer, insbesondere programmtechnischer Mittel eine automatische Beurteilung der Teigqualität erlaubt. Fällt eine aufgezeichnete Kurve aus dem in Figur 4 mit mittlerer Referenzkurve eingezeichneten Toleranzband, so liegt Teig-Ausschuß vor, und es können entsprechende Maßnahmen ergriffen werden, die Ausgabe eines Alarmsignals und/oder Stoppen der Teigförderung. Ferner kann eine im Steuerungsrechner implementierte Regelung (vgl. Figuren 5 und 6) durch Beeinflussung der Teigbearbeitungs-Stellglieder dafür sorgen, dass das als Istzustand ermittelte Kraft-Weg-Diagramm möglichst an die Referenzkurve angenähert ist.

Gemäß Figur 5 sind die Hauptprozesse "Dosieren", "Kneten", "Gären", "Teig formen/-aufbereiten" "Gären" und "Backen" zeitlich hintereinander ablaufend angeordnet. Ihre Koordination und Synchronisation aufeinander wird mittels einer Regelungseinrichtung sowie einer übergeordneten Prozeßleitsteuerung mit Führungsgrößenvorgabe für die Mehrzahl der Prozesse als Regelungsstrecken bewerkstelligt. Derart erstreckte Regelungsstrukturen sind an sich bekannt (vgl. Otto Föllinger "Regelungstechnik", 8. Auflage, Hüthig Buchverlag Heidelberg, dort insbes. Seite 285 "Mehrgrößenregelung"), nicht jedoch im Zusammenhang bzw. in der Anwendung mit bzw. auf Teigverarbeitungs- und Bäckereitechnologie. Erfindungsgemäß umfaßt die Istwerterfassung wenigstens bei dem Prozeß "Teigformen/-aufbereiten" den Einsatz ein oder mehrerer Meßanordnungen gemäß Figuren 1-3 zur Ermittlung der Teigfestigkeit und -qualität. Der Soll-/Istwertvergleich kann auf dem Toleranzband gemäß Figur 4 basieren.

Gemäß Figur 6 besteht der Hauptprozeß einer maschinellen Brezellinie aus üblichen Unterschritten bzw. Subprozessen der Teigverarbeitung, die dem Fachmann an sich bekannt sind. Erfindungsgemäß werden die einzelnen Subprozesse mittels eines vernetzten Regelungs- und Steuerungssystems mit lokal verteilten, gegebenenfalls in sich autonomem Einzelregelungen (die in sich wiederum als Einfach- oder Mehrgrößenregler ausgebildet sein können) aufeinander koordiniert, synchronisiert bzw. abgestimmt. Eine zentrale Rolle dafür spielt die gezeichnete Prozeßleitsteuerung mit Führungsgrößenvorgabe, welche über das Kommunikationssystem "Feldbus" mit den verteilten, vorzugsweise mit digitalen Prozessoren realisierten Regelungsintelligenzen LR kommuniziert. Derart vernetzte Systeme mit verteilter Steuerung sind an sich bekannt (vgl. z. B. Wolfhard Lawrenz "CAN CONTROLLER AREA NETWORK", Hüthig Verlag Heidelberg, insbes. Seite 85). Zweckmäßig werden derartige Regelungs- und Steuerungssysteme mit über Feldbus vernetzte, lokale Regelungsprozessoren zum Betrieb einer maschinellen Teigformlinie, insbesondere Brezellinie, eingesetzt. Erfindungsgemäß wird wenigstens im lokalen Regler des Subprozesses "Teig-Vorformen" ein Soll-/Istwertvergleich auf der Basis des in Figur 4 dargestellten Toleranzbandes für den Elastizitätsmodul vorgenommen, wobei eine Meßanordnung gemäß Figuren 1-3 am Eingang des Vorformers Anwendung finden kann. Dadurch läßt sich eine Eingangs-Qualitätskontrolle für die Teigmasse vornehmen, die aus einem vorgeschalteten Teigbildungsprozeß mit Dosieren, Kneten und gegebenenfalls Gären gewonnen wurde. Sollte die Qualität der Teigmasse gemäß Ausgangsergebnis der Teigfestigkeitsmessung aus dem Toleranzband gemäß Figur 4 fallen, können entsprechende Vorsichtsund Sicherheitsmaßnahmen ergriffen werden. Insbesondere kann der lokale Regler für das Teig-Vorformen über den Feldbus eine Warnnachricht an die Prozeßleitsteuerung und/oder die nachfolgenden lokalen Regler übermitteln, woraufhin diese die ihnen zugeordneten Stellglieder gemäß einer vorliegenden Qualitätssicherungsspezifikation ansteuern, beispielsweise anhalten oder abschalten. Über die Prozeßleitsteuerung und das Mensch-Maschine-Interface MMI oder auch über das Web-Interface kann ein Alarm an Bedienpersonen ausgegeben werden.

### Bezugszeichenliste

- 1: Gestell
- 2: Führungsstange
- 2a: Teig-Förderband
- 3: Führungshülse
- 4: Trägerwagen
- 4a: Vertikalwange
- 5: Basisplatte
- 5a: Verschraubung
- 5b: Befestigungselement
- 6: Meßgerät
- 7: Mitnehmerelement
- 7a: Klemmplatte
- 8: Rückstellantrieb
- 9: Querverstellführung
- 10: Förderrichtung
- 11: Teig
- 12: Druckplattensensor
- 13: Hubbewegung
- 14: Stellorgan
- Vk: Geschwindigkeit
- X: Eintauchtiefe
- F: Widerstands- bzw. Reaktionskraft

## Patentansprüche

1. Messverfahren zur Bestimmung und Überwachung der Teigqualität für eine automatische Teigbearbeitung, wozu mittels mindestens eines den Teig (11) kontaktierenden Druck- oder Kraft-Sensors (12) eine vom Teig (11) ausgeübte Reaktionskraft (F) oder Reaktionsauslenkung als Maß für dessen Festigkeit gemessen und entsprechende Messdaten oder -signale einer Steuerung für die Teigbearbeitung zugeleitet werden, **dadurch gekennzeichnet, dass** mehrere zeitlich und örtlich diskrete Messungen an fertig bereitetem Teig (11) ausgeführt werden, der als kontinuierliches Teigband oder als einzelne Teigstücke ausgebildet ist, wobei das Teigband oder die Teigstücke (11) zumindest zeit- oder taktweise über ein oder mehrere Fördermittel (2a) in einer Förderrichtung (10) bewegt werden, und der Sensor (12) synchron zur Fördergeschwindigkeit mit dem Teig (11) in Kontakt gebracht wird.

2. Messverfahren nach Anspruch 1, **gekennzeichnet durch** eine oder mehrere wiederholt und/oder zeitlich beabstandet ausgeführte Messungen am Teigband oder an dem jeweiligen Teigstück (11).

3. Messverfahren nach Anspruch 1 oder 2, **dadurch gekennzeichnet, dass** Messzyklen wiederholt werden, innerhalb welcher jeweils eine oder mehrere Messungen am Teigband oder Teigstück (11), gegebenenfalls hintereinander und/oder an lokal unterschiedlichen Bereichen, durchgeführt werden.

4. Messverfahren nach einem der vorangehenden Ansprüche, **dadurch gekennzeichnet, dass** der Sensor (12) zum Kontaktieren des bewegten Teigs (11) synchron zu dessen Fördergeschwindigkeit mitbewegt wird.

5. Messverfahren nach Anspruch 4, **dadurch gekennzeichnet, dass** zu Messbeginn oder am Anfang eines Messzyklusses der Sensor (12) mit dem oder den sich bewegenden Fördermitteln (2a) mechanisch gekoppelt, mit dem geförderten Teig (11) in Berührung gebracht und mitbewegt sowie zum Mess- oder Zyklusende wieder entkoppelt, vom Teig (11) entfernt und in eine Ausgangsstellung zurückbewegt oder in eine nächste Messstellung weiterbewegt wird.

6. Messverfahren nach einem der vorangehenden Ansprüche, **dadurch gekennzeichnet, dass** in Wirkungsverbindung mit dem Sensor (12) eine zunehmende Auslenkung (X) von Teigmasse bewirkt und dabei der Verlauf (Fig.4) der Kraft (F) oder des Drucks in Abhängigkeit vom Auslenkungsweg (X) oder umgekehrt der Auslenkungsweg (X) in Abhängigkeit vom Verlauf der Kraft (F) oder des Drucks zu Steuerungs- oder Regelungszwecken ermittelt, gespeichert und ausgewertet werden.

7. Messverfahren nach Anspruch 6, **dadurch gekennzeichnet, dass** zur Auslenkung (X) der Teigmasse der Sensor (12) in die Teigberfläche eingedrückt wird.

8. Messverfahren nach einem der vorangehenden Ansprüche, **dadurch gekennzeichnet, dass** vor oder nach einer Messung, gegebenenfalls innerhalb eines Messzyklusses, der Sensor (12) schräg oder quer zur Förderrichtung (10) bewegt und damit in eine nächste Messstellung verstellt wird.

9. Messverfahren nach einem der vorangehenden Ansprüche, **gekennzeichnet durch** die zeitgleiche Verwendung mehrerer Sensoren (12) zu parallelen Messungen an lokal unterschiedlichen Bereichen des Teigbands oder Teigstücks (11).

10. Verfahren zur Regelung und/oder Steuerung eines Prozesses des Teigformens/-aufbereitens mit mehreren Subprozessen wie Vorformen, Kalibrieren, Ausrollen, Querwalzen, Schneiden in einzelne Teigstücke, Gären, Wickeln, Biegen und/oder Ineinanderschlingen einzelner Teigstücke, wobei die genannten Subprozesse einander vor- und/oder nachgeordnet und durch jeweils zugeordnete Teigbearbeitungsgeräte oder -maschinen als Stellglieder beeinflußt werden, und wenigstens einer der Subprozesse mit Soll-/Istwervergleichen geregelt wird, insbesondere unter Verwendung des Messverfahrens nach einem der vorangehenden Ansprüche, **dadurch gekennzeichnet, dass** bei wenigstens einem der Subprozesse, vorzugsweise zu Beginn und/oder am Eingang des Prozesses oder beim als ersten ablaufenden Subprozess, der Elastizitätsmodul des Teiges (11) erfaßt und einem Soll-/Istwertvergleich zur Regelung wenigstens dieses Subprozesses zugeführt wird.

11. Verfahren nach Anspruch 10, **dadurch gekennzeichnet, dass** zwei oder mehr der Subprozesse gemeinsam überwacht, gesteuert und mit Soll-/Istwertvergleichen geregelt werden, wobei die jeweiligen Überwachungssignale in einer gemeinsamen Leitstelle verarbeitet, und/oder Steuerungsund/oder Regelungs-Führungsgrößen von dieser Leitstelle aus für die wenigstens zwei Subprozesssteuerungen oder -regelungen koordiniert und/oder synchronisiert generiert und vorgegeben werden,

12. Verfahren nach Anspruch 11, **dadurch gekennzeichnet, dass** mehrere Teig-Elastizitätsmodul-Erfassungen über die Reihe der Subprozesse verteilt durchgeführt und für mehrere Soll-/Istwerterfassungen verwendet werden.

13. Verfahren nach Anspruch 11 oder 12, **dadurch gekennzeichnet, dass** die Subprozesse jeweils lokal mit eigener Soll-/Istwert-Erfassung geregelt werden.

14. Verfahren nach einem der vorangehenden Ansprüche, **dadurch gekennzeichnet, dass** unmittelbar nach Abschluß eines vorgeschalteten Teigbildungsprozesses gegebenenfalls mit Subprozessen wie Teigkneten und/oder -mischen und gegebenenfalls nach einer zusätzlichen Vorgärphase die Materialeigenschaften dieses Teiges erfaßt und zur Bildung von Führungsgrößen für die Subprozesse des nachgeordneten Prozesses des Teigformens/-aufbereitens verwendet werden.

15. Verfahren nach Anspruch 14, **dadurch gekennzeichnet, dass** über einen vorbestimmten Zeitraum ein oder mehrere Materialeigenschaften des Teiges (11) jeweils unmittelbar nach Beendigung des Prozesses "Teigbilden" und vor Beginn des Prozesses "Teigformen/-aufbereiten" erfaßt und als Kennlinie gespeichert werden, und diese Kennlinie später zur Berechnung von Führungsgrößen für den Prozess "Teigformen/-aufbereiten" verwendet wird.

16. Messanordnung zur Bestimmung und Überwachung der Teigqualität für eine automatische Teigbearbeitung, zur Durchführung des Messverfahrens nach einem der Ansprüche 1 bis 9, mit wenigstens einem Druck- oder Kraft-Sensor (12), der zur mechanischen Einwirkung auf den Teig (11) angeordnet, zur Messung einer Reaktionskraft (F) oder -auslenkung (X) des Teigs (11) ausgebildet und ausgangsseitig mit einer Steuerung für die Teigbearbeitung verbunden oder verbindbar ist, **dadurch gekennzeichnet, dass** der Sensor (12) mit einem von der Steuerung kontrollierten, motorisch angetriebenen Stellorgan (14) verbunden ist, welches eine räumliche Verstellung des Sensors (12) ermöglicht, um diesen mit dem Teig (11) in und außer Eingriff zu bringen, und das aus Sensor (12) und Stellorgan (14) gebildete Messgerät (6) von von der Steuerung kontrollierten Kopplungsmitteln (2,3,4,5,7,8) zur Synchronisation mit der Bewegung (10) einer Teig-Fördereinrichtung (2a) erfasst ist, um den Sensor (12) und/oder dessen Stellorgan (14) mit der Förderbewegung (10) des von der Fördereinrichtung (2a) erfassten Teigs (11) zu synchronisieren und/oder mitzubewegen.

17. Messanordnung nach Anspruch 16, **dadurch gekennzeichnet, dass** zur Mitbewegung des Sensors (12) mit der Förderbewegung (10) des Teigs (11) die Kopplungsmittel (2,3,4,5,7,8) eine parallel zur Fördereinrichtung (2a) verlaufende Parallelführung (2,3) und einen davon spurgeführten, von der Fördereinrichtung (2a) mitbewegbaren Trägerwagen (4) aufweisen, worauf das aus Sensor (12) und Stellorgan (14) gebildete Messgerät (6) angebracht ist.

18. Messanordnung nach Anspruch 16 oder 17, **dadurch gekennzeichnet, dass** zur Verstellung des Messgeräts schräg oder quer zur Förderrichtung eine entsprechend verlaufende Querverstellführung (9) angeordnet ist, von der das Messgerät (6)erfasst oder erfassbar ist.

19. Messanordnung nach Ansprüche 17 und 18, **dadurch gekennzeichnet, dass** die Querverstellführung (9) an oder auf dem Trägerwagen (4) angebaut und so mit der Fördereinrichtung (2a) mitbewegbar ist.

20. Messanordnung nach einem der vorangehenden Ansprüche, **dadurch gekennzeichnet, dass** die Kopplungsmittel (2,3,4,5,7,8) als Unterlage für die Fördereinrichtung (2a) und/oder den Teig (11) eine Basisplatte (5) aufweisen, welche dem Sensor (12) gegenüberliegend angeordnet ist und/oder gehalten wird.

21. Messanordnung nach Anspruch 17 und 20, **dadurch gekennzeichnet, dass** die Basisplatte (5) mit dem Trägerwagen (4) fest verbunden ist.

22. Messanordnung nach einem der vorangehenden Ansprüche, **dadurch gekennzeichnet, dass** die Kopplungsmittel (2,3,4,5,7,8) ein oder mehrere Mitnehmerelemente (7) aufweisen, die von der Steuerung betätigbar und mit der Fördereinrichtung (2a) in Eingriff bringbar sind.

23. Messanordnung nach Anspruch 22 und 20 oder 21, **dadurch gekennzeichnet, dass** das oder die Mitnehmerelemente (7) als mechanisch bewegbare Klemmorgane (7a) ausgebildet sind, die über die Steuerung zur Basisplatte (5) hin verstellbar sind.

24. Messanordnung nach einem der vorangehenden Ansprüche, **dadurch gekennzeichnet, dass** die Kopplungsmittel (2,3,4,5,7,8) einen ortsfest abgestützten und am Messgerät (6), dem Trägerwagen (4) oder an der Basisplatte (5) angreifenden sowie von der Steuerung kontrollierbaren Rückstellantrieb (8) aufweisen, der entgegen der Förderrichtung (10) zur Verstellung des Messgeräts (6) in eine Ausgangsstellung betätigbar ist.

25. Anordnung zur Regelung und/oder Steuerung eines Prozesses des Teigformens/-aufbereitens, der Subprozesse wie Vorformen, Kalibrieren, Ausrollen, Querwalzen, Schneiden in einzelne Teigstücke (11), Gären, Wickeln, Biegen und/oder Ineinanderschlingen einzelner Teigstücke (11) umfaßt, mit einem oder mehreren Teigverarbeitungsgeräten oder -maschinen als das Teigformen/-aufbereiten oder die Subprozesse bewirkende Stellglieder, mit einer diesen gemeinsamen Steuerungs- und Regeleinrichtung zur Einstellung und Ansteuerung eines oder mehrerer der Stellglieder, und mit mindestens einer Messanordnung zur Bildung einer Istwert-Sensorik (6,12) für die Regeleinrichtung, **dadurch gekennzeichnet, dass** bei wenigstens einem der Subprozesse, vorzugsweise zu Beginn und/oder am Eingang des Prozesses oder beim als ersten ablaufenden Subprozess, eine Messanordnung (Fig.1-3) zur Ermittlung der Teig-Festigkeit angeordnet, in synchronisierter Wirkungsverbindung mit dem geförderten Teig (11) dieses Subprozesses setzbar und ausgangsseitig mit der Regeleinrichtung verbindbar oder verbunden ist, die programm- und/oder schaltungstechnisch zur Ermittlung eines Teig-Elastizitätsmoduls (Fig.4) und zu dessen Auswertung und Vergleich mit einem Sollwert ausgebildet ist.

26. Anordnung nach Anspruch 25, **dadurch gekennzeichnet, dass** zwischen einem dem vorausgehenden Teigbildungsprozess und dem diesen nachfolgenden Prozess des Teigformens/-aufbereitens eine von der Regeleinrichtung über ein eigens zugeordnetes Stellglied kontrollierbare Teig-Fördereinrichtung angeordnet ist, und die Regeleinrichtung ein derart programm- und/oder schaltungstechnisch eingerichtetes Steuerungsmodul aufweist, dass abhängig von am Ende des ersten Prozesses erfassten Teig-Istwerten die Teig-Fördereinrichtung (2a) zum nachfolgenden Prozess aktivierbar ist.

27. Anordnung nach Anspruch 25 oder 26, **dadurch gekennzeichnet, dass** zwei oder mehr der die Subprozesse bewirkenden oder beeinflussenden Stellglieder zu ihrer Ansteuerung oder Einstellung mit der gemeinsamen Steuerungs- oder Regeleinrichtung verbunden sind, welche eingangs- und ausgangsseitig mit einer Prozeßleitsteuerung verbunden ist, die zur Erzeugung und Ausgabe von Führungsgrößen, koordiniert und/oder synchronisiert für zwei oder mehr der Subprozesse, an die Steuerungs- oder Regeleinrichtung eingerichtet und/oder programmiert ist.

28. Anordnung nach einem der vorangehenden Ansprüche, **dadurch gekennzeichnet, dass** die Regeleinrichtung mit wenigstens zwei der Subprozesse als Regelstrecken über eine jeweilige Istwert-Sensorik (6,12) für Teigeigenschaften gekoppelt ist und jeweils einem Subprozess zugeordnete Soll/Istwert-Vergleichsglieder aufweist, die je mit einer der Istwert-Sensoriken (6,12) verbunden sind.

29. Anordnung nach einem der vorangehenden Ansprüche, **dadurch gekennzeichnet, dass** die Regeleinrichtung eine Mehrzahl lokaler Regler jeweils mit eigenem Soll-/lstwert-Vergleichsglied umfaßt, das einem der Subprozesse zugeordnet ist, und die lokalen Regler über ein Bussystem mit der Prozessleitsteuerung kommunizieren.
